(19) Europäisches Patentamt
European Patent Office
Office européen des brevets

(11) **EP 4 467 210 B1**

(12) **EUROPEAN PATENT SPECIFICATION**

(45) Date of publication and mention
of the grant of the patent:
**01.04.2026 Bulletin 2026/14**

(21) Application number: **22961046.4**

(22) Date of filing: **30.09.2022**

(51) International Patent Classification (IPC):
*A43D 1/02* (2006.01)        *A61B 5/11* (2006.01)
*G01C 22/00* (2006.01)       *A61B 5/00* (2006.01)

(52) Cooperative Patent Classification (CPC):
**A61B 5/1123; A61B 5/1038; A61B 5/112;
A61B 5/7264; G01C 22/006; G16H 20/30**

(86) International application number:
**PCT/JP2022/036868**

(87) International publication number:
**WO 2024/069987 (04.04.2024 Gazette 2024/14)**

(54) **RUNNING STYLE ANALYSIS DEVICE, RUNNING STYLE ANALYSIS METHOD, AND RUNNING STYLE ANALYSIS PROGRAM**

VORRICHTUNG ZUR LAUFSTILANALYSE, VERFAHREN ZUR LAUFSTILANALYSE UND PROGRAMM ZUR LAUFSTILANALYSE

DISPOSITIF D'ANALYSE DE STYLE DE COURSE, PROCÉDÉ D'ANALYSE DE STYLE DE COURSE ET PROGRAMME D'ANALYSE DE STYLE DE COURSE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.11.2024 Bulletin 2024/48**

(73) Proprietor: **ASICS Corporation
Kobe-shi, Hyogo 650-8555 (JP)**

(72) Inventors:
• **SAKAGUCHI, Masanori
Kobe-shi Hyogo 650-8555 (JP)**
• **HIRAKAWA, Nao
Kobe-shi Hyogo 650-8555 (JP)**
• **NAKAYA, Seigo
Kobe-shi Hyogo 650-8555 (JP)**
• **TANIGUCHI, Norihiko
Kobe-shi Hyogo 650-8555 (JP)**

(74) Representative: **Marks & Clerk LLP
15 Fetter Lane
London EC4A 1BW (GB)**

(56) References cited:
**WO-A1-2018/033965     JP-A- 2016 214 499
JP-B2- 4 856 427**

• **ANONYMOUS: "Metaspeed: Which is better, Sky+ or Edge+?", TOMO.RUN, 24 September 2022 (2022-09-24), pages 1 - 18, XP093151825, Retrieved from the Internet <URL:https://tomo.run/metaspeed-sky-edge> [retrieved on 20240415]**

# EP 4 467 210 B1

**Description**

BACKGROUND

1. Technical Field

The present disclosure relates to a technology for analyzing running styles of marathon runners.

2. Background Information

In long-distance running such as marathons, "high cadence running" and "long stride running" are known as running styles of runners. In general, the former is considered as a running style with relatively high step frequency (which is the number of steps per unit time and also referred to as "cadence") and relatively short step length (which is the length of one step and also referred to as "stride length"), and the latter as a running style with relatively low step frequency and relatively long step length; however, there is no clear definition.

In recent years, running shoes suitable for the high cadence running and those suitable for the long stride running have been developed respectively. Therefore, a runner may be able to select more suitable shoes by knowing whether his or her running style is the high cadence type or the long stride type (see Patent Literature 1, for example).

RELATED-ART LITERATURE

PATENT LITERATURE

Patent Literature 1: Japanese Patent No. 4856427

JP 2016 214499 A discloses providing of guidance on at least one selected from a group consisting of a speed, a pitch, and a stride of a user's movement, based on a movement characteristic representing a relation among a combination of the speed, the pitch, and the stride during the user's movement.

However, except in cases where the tendency of the step frequency or the step length is particularly pronounced, there have been conventionally no clear criteria for determining whether the running style of a runner falls under the high cadence type or the long stride type. Accordingly, such determining has had to rely on subjective judgment.

Under such circumstances, as a result of analyzing the running records of a large number of runners, the inventors have found a method for distinguishing between the both types, with objective criteria based on the running style tendency.

SUMMARY

The present disclosure has been made in view of such an issue, and a purpose thereof is to provide a technology for analyzing the running style of a runner.

To solve the problem above, a running style analysis device according to one embodiment of the present disclosure includes: a step frequency acquirer that acquires, with regard to running of a subject, a slope of a step frequency change with respect to a change in running speed; a step length acquirer that acquires, with regard to running of the subject, a slope of a step length change with respect to a change in running speed; a judgment unit that calculates a principal component score from the slope of a step frequency change and the slope of a step length change of the subject, based on a principal component analysis model generated in advance based on measurement values of multiple runners and that makes, based on the principal component score thus calculated, judgment as to which of multiple running style types, including a long stride type and a high cadence type, running of the subject falls under; and a result output unit that outputs a result of the judgment.

Another embodiment of the present disclosure is a computer-implemented running style analysis method. This method includes:
acquiring, with regard to running of a subject, a slope of a step frequency change with respect to a change in running speed; acquiring, with regard to running of the subject, a slope of a step length change with respect to a change in running speed; calculating a principal component score from the slope of a step frequency change and the slope of a step length change of the subject, based on a principal component analysis model generated in advance based on measurement values of multiple runners; making, based on the principal component score thus calculated, judgment as to which of multiple running style types, including a long stride type and a high cadence type, running of the subject falls under; and outputting a result of the judgment.

Optional combinations of the aforementioned constituting elements, and implementation of the present disclosure, including the constituting elements and expressions, in the form of methods, apparatuses, programs, transitory or non-transitory storage medium storing programs, or systems may also be practiced as additional modes of the present disclosure.

BRIEF DESCRIPTION OF DRAWINGS

[Fig. 1] Fig. 1 is a diagram that illustrates a basic configuration of a running style analysis system.
[Figs. 2A and 2B] Figs. 2A and 2B are diagrams in which changes in step length and changes in step frequency are compared between the long stride type and the high cadence type in the same running speed range.
[Fig. 3] Fig. 3 is a diagram that illustrates relationships between the slope of a step frequency change and the slope of a step length change for multiple running speeds.
[Fig. 4] Fig. 4 is a functional block diagram that shows a basic configuration of a user terminal.
[Fig. 5] Fig. 5 is a functional block diagram that shows a basic configuration of a running style analysis server.
[Fig. 6] Fig. 6 is a diagram that shows a distribution of principal components obtained by principal component analysis.
[Fig. 7] Fig. 7 is a diagram that shows a distribution of a first principal component obtained by principal component analysis.
[Fig. 8] Fig. 8 is a flowchart that shows basic processing performed in the running style analysis server.
[Fig. 9] Fig. 9 is a diagram that shows relationships between the distribution of the first principal component obtained by principal component analysis and ranges of running style types.

DETAILED DESCRIPTION

In the following, the present disclosure will be described based on preferred embodiments with reference to each drawing. In the embodiments and modifications, like reference characters denote like or corresponding constituting elements, and the repetitive description will be omitted as appropriate. In each drawing, parts less important in describing the embodiments may be omitted.

The "running style analysis device" in the claims may be implemented by a combination of a server and a server program running on a web server or in the cloud, or by a combination of a device, such as a smartphone, a tablet, any other information terminal, or a personal computer, and a program running on such a device. Alternatively, the running style analysis device may be implemented by a combination of a wearable device with various sensors embedded therein, and a program running on such a wearable device. The following embodiments describe examples of a "running style analysis device" implemented by a combination of a server and a server program, and a running style analysis system that includes a user's terminal or wearable device.

First Embodiment

In the present embodiment, it is premised that a user, who is a runner and wants to know whether running shoes (hereinafter, simply referred to as "shoes") for high cadence running or shoes for long stride running are suitable for him or her, performs running style analysis by himself or herself. First, the user wears various wearable devices during running, acquires information necessary for analysis with various sensors, and transmits the information to the user's terminal. Thereafter, the user transmits the information from the terminal to a server and obtains analysis results from the server.

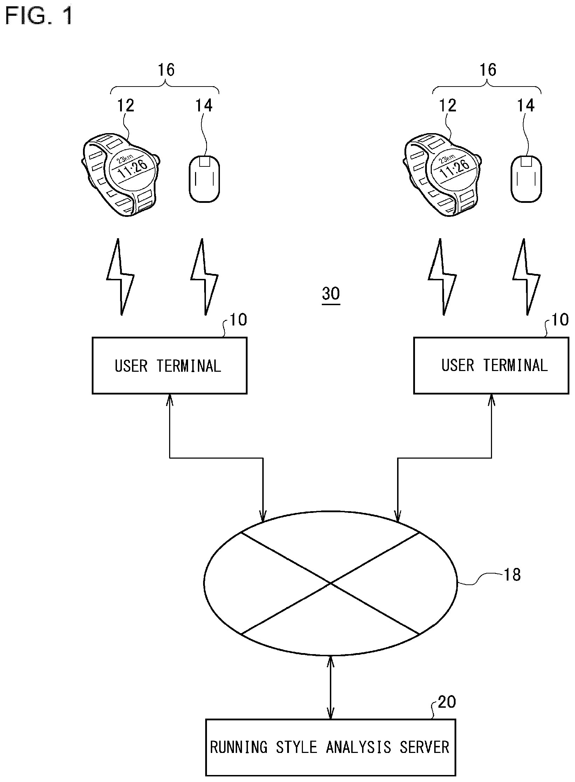

Fig. 1 illustrates a basic configuration of a running style analysis system. A running style analysis system 30 is constituted by a user terminal 10, a wearable device 16, and a running style analysis server 20, for example. A user performs running while wearing a wearable device 16, such as a running watch 12 and a motion sensor 14, on an arm or the waist and acquires various detection data with the running watch 12 and the motion sensor 14. The running watch 12 and the motion sensor 14 include sensors such as a positioning module and a 9-axis motion sensor. The running speed is acquired based on the relationship between time information and position information detected by the positioning module, and the step frequency is acquired based on information detected by the 9-axis motion sensor. Also, the step length is acquired based on the running distance measured by the positioning module and the step frequency (step length = running distance ÷ step frequency). A running log acquired by the user terminal 10 is transmitted to the running style analysis server 20 via a network 18. Accordingly, the running style is analyzed by the running style analysis server 20, and which of multiple running styles, including the high cadence type and the long stride type, the running style corresponds to is judged.

In a modification, instead of a wearable device 16, a positioning module or a motion sensor built into a smartphone as the user terminal 10 may be used. In another modification, as data indicating the running state of the subject, running speed data and step frequency data may be acquired from images captured by a high-speed camera using a technology such as motion capture or may be acquired by detecting floor reaction force using a force plate. In such a case, an operator other than the user (e.g., a salesperson in a store) may operate the user terminal 10 to acquire the running state data for the subject and allow the running style analysis server 20 to perform running style analysis.

The "step frequency" information is, for example, a numerical value in units of the number of steps per second (Hz) or the number of steps per minute (spm). When a runner whose marathon completion time is within 3 hours and 30 minutes runs at race pace, the number of steps per minute generally falls within the range of 175 to 205 spm on average. Also, the "step length" information is an average step length (m), which is obtained by dividing the running distance per minute by the number of steps per minute. In the present embodiment, the step frequency data and step length data to be analyzed are not limited to those of running speeds of advanced runners whose marathon completion times are within 3 hours, for example, and may be data of running speeds corresponding to the completion times of 3 hours or more, such as within 4 hours, as long as the relationships between the slope of a step frequency change and the slope of a step length change for multiple running speeds, which will be described later, can be detected.

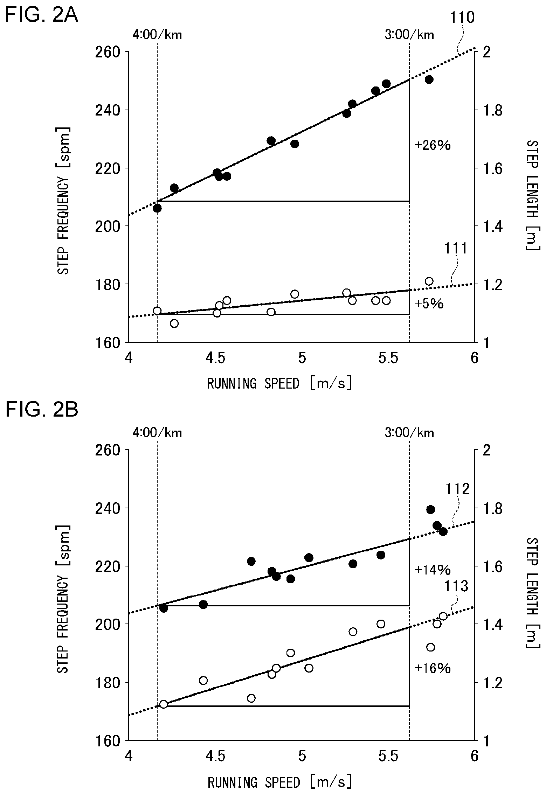

Figs. 2A and 2B are diagrams in which changes in step length and changes in step frequency are compared between the long stride type and the high cadence type in the same running speed range. Fig. 2A is a scatter plot that illustrates the relationship between the running speed and the step length and the relationship between the running speed and the step frequency of a long stride type runner, whose personal best marathon completion time is 2 hours 36 minutes 7 seconds. Fig. 2B is a scatter plot that illustrates the relationship between the running speed and the step length and the relationship between the running speed and the step frequency of a high cadence type runner, whose personal best marathon completion time is 2 hours 40 minutes 0 seconds. The horizontal axis represents the running speed [m/s], and the vertical axis represents the step length [m] or step frequency (number of steps per minute) [spm]. In Figs. 2A and 2B, the step length (filled circle marks) and the step frequency (open circle marks) are plotted for the case of running in the range of 4.0 to 6.0 m/s, including 4.17 m/s (4 min/km pace) and about 5.56 m/s (3 min/km pace).

In the case of the long stride type runner shown in Fig. 2A, the step length increases greatly by a wide range of about 0.55 m, from about 1.45 m to about 2 m, in proportion to the increase in running speed. The slope of a step length change 110, which is a regression line indicating the increase in step length with respect to the increase in running speed, is relatively large. In particular, with respect to the increase in running speed from 4.17 m/s (4 min/km pace) to about 5.56 m/s (3 min/km pace), the step length increases by +0.39 m (about 26%) from 1.49 m to 1.88 m.

In contrast, the step frequency (number of steps per minute) of the long stride type runner gradually increases by a narrow range from 169 spm to 183 spm in proportion to the increase in running speed. The slope of a step frequency change 111, which is a regression line indicating the increase in step frequency with respect to the increase in running speed, is slight and nearly flat. In particular, with respect to the increase in running speed from 4.17 m/s (4 min/km pace) to about 5.56 m/s (3 min/km pace), the step frequency (number of steps per minute) increases by only +8 spm (about 5%) from 169 spm to 177 spm.

Meanwhile, in the case of the high cadence type runner shown in Fig. 2B, the step length increases by a range of about 0.3 m, from about 1.45 m to about 1.75 m, in proportion to the increase in running speed. The slope of a step length change 112, which is a regression line indicating the increase in step length with respect to the increase in running speed, is smaller than that of the long stride type. In particular, with respect to the increase in running speed from 4.17 m/s (4 min/km pace) to about 5.56 m/s (3 min/km pace), the step length increases by only +0.2 m (about 14%) from 1.48 m to 1.68 m.

In contrast, the step frequency (number of steps per minute) of the high cadence type runner increases greatly by a wide range from 168 spm to 205 spm in proportion to the increase in running speed. The slope of a step frequency change 113, which is a regression line indicating the increase in step frequency with respect to the increase in running speed, is greater than that of the long stride type. In particular, with respect to the increase in running speed from 4.17 m/s (4 min/km pace) to about 5.56 m/s (3 min/km pace), the step frequency (number of steps per minute) increases by +28 spm (about 16%) from 170 spm to 198 spm.

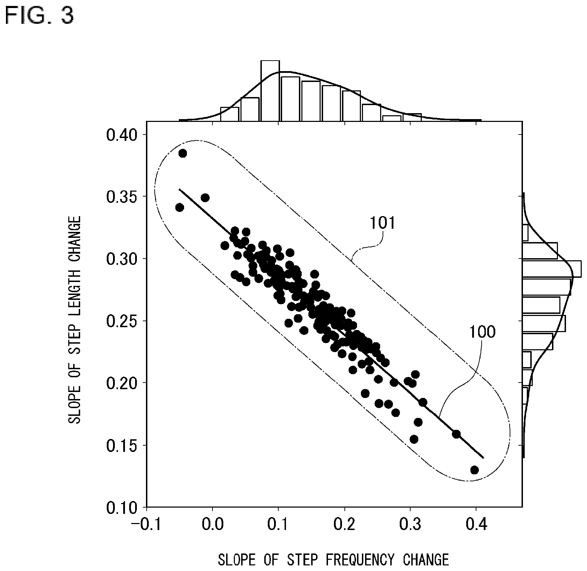

Fig. 3 is a diagram that illustrates relationships between the slope of a step frequency change and the slope of a step length change with respect to a change in running speed of multiple runners. When the slope of the step frequency (number of steps per minute) change is plotted on the horizontal axis and the slope of the step length change is plotted on the vertical axis, a negative correlation distributed in a region 101 inclined toward the lower right can be seen, as shown in the figure. That is, it can be seen that the slope of the step length change tends to be smaller for a runner with a larger slope of the step frequency change and tends to be larger for a runner with a smaller slope of the step frequency change. When regression analysis is performed on the relationships between the slope of the step frequency change and the slope of the step length change, a regression line 100 having a negative slope is obtained as shown in the figure.

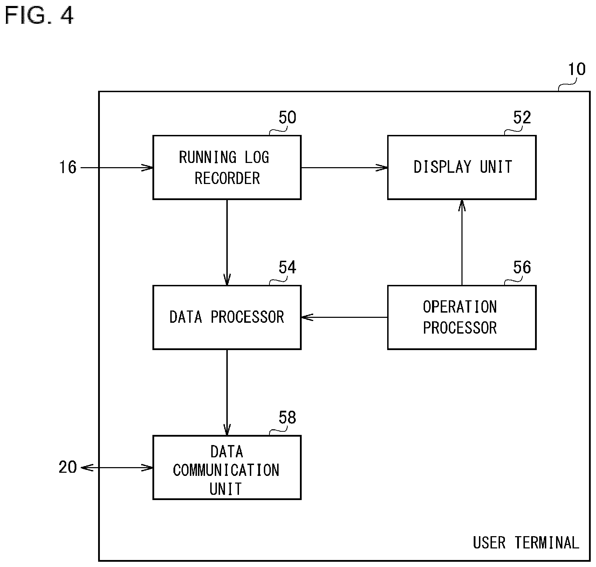

Fig. 4 is a functional block diagram that shows a basic configuration of a user terminal. Fig. 4 is a block diagram featuring the functions, and these functional blocks may be implemented in a variety of forms by hardware, software, or a combination thereof. The user terminal 10 may be, for example, a device, such as a smartphone, a tablet terminal, any other information terminal, or a personal computer, in terms of hardware. The user terminal 10 includes at least each function of a running log recorder 50, a display unit 52, a data processor 54, an operation processor 56, and a data communication unit 58. The user terminal 10 is constituted by, for example, a CPU (Central Processing Unit), ROM (Read Only Memory), RAM (Random Access Memory), touch panel, communication module, and the like, in terms of hardware. As a modification, each function of the user terminal 10 shown in Fig. 4 may be built into a wearable device 16 so as to be implemented as an integrated device.

The running log recorder 50 acquires various detection data from a wearable device 16 via a communication module such as short-range wireless communication and records the data as a running log. The detection data acquired from a wearable device 16 include position information received from a satellite positioning system, such as the GPS (Global Positioning System), information indicating the acquisition date and time thereof, and information on the step frequency (the number of steps per unit time, such as per minute). Based on the detection data acquired from a wearable device 16, the running log recorder 50 records, as a running log, information such as the running time, running distance, running speed for each predetermined distance or each predetermined time, and step frequency, in a predetermined storage area.

The operation processor 56 accepts operation input for an instruction from the user. Based on the user's instruction via the operation processor 56, the display unit 52 displays, on a screen, a running log recorded by the running log recorder 50. Also, based on the user's instruction via the operation processor 56, the data processor 54 extracts data of step frequency at multiple running speeds from running logs recorded by the running log recorder 50 and transmits the extracted data as the subject's running logs to the running style analysis server 20 via the data communication unit 58. In a modification, the data processor 54 may transmit all the running logs to the running style analysis server 20 via the data communication unit 58, and necessary data may be extracted on the running style analysis server 20 side. Also, the step length data may be generated by dividing the running distance by the step frequency and included in the running log.

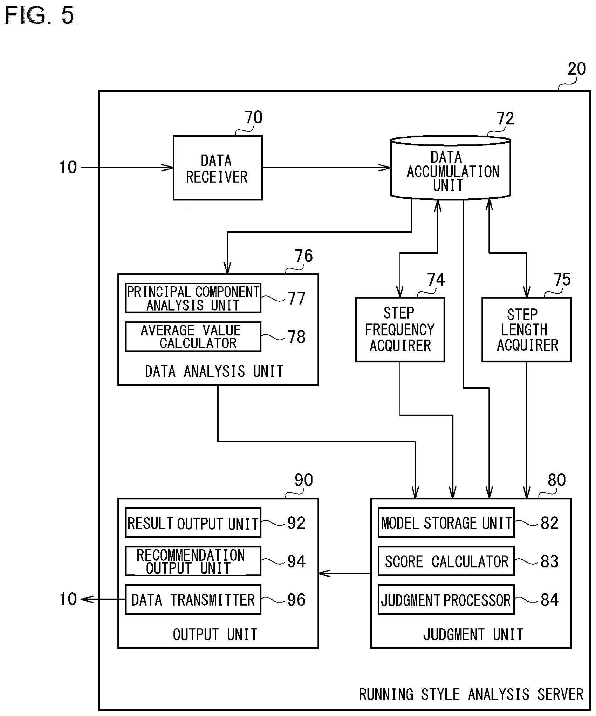

Fig. 5 is a functional block diagram that shows a basic configuration of the running style analysis server. Fig. 5 is a block diagram featuring the functions, and these functional blocks may be implemented in a variety of forms by hardware, software, or a combination thereof. The running style analysis server 20 may be, for example, a server computer in terms of hardware. The running style analysis server 20 includes at least each function of a data receiver 70, a data accumulation unit 72, a step frequency acquirer 74, a step length acquirer 75, a data analysis unit 76, a judgment unit 80, and an output unit 90. The running style analysis server 20 is constituted by, for example, a CPU, ROM, RAM, communication module, and the like, in terms of hardware.

The data receiver 70 receives, from the user terminal 10, running speed data and step frequency data included in a running log of the subject and stores those data in the data accumulation unit 72. The data accumulation unit 72 accumulates a data group of step frequency and step length based on the running logs of a large number of runners measured in the past. The data group accumulated in the data accumulation unit 72 is subjected to principal component analysis and stored as a principal component analysis model in the judgment unit 80. The principal component analysis model is used to judge whether the runner's running style is the high cadence type or the long stride type, based on a running log newly acquired. Although the "step frequency" in Fig. 2 mainly indicates the number of steps per minute (spm), the number of steps per second (Hz), obtained by dividing the number of steps per minute by 60, may also be used as the numerical value of "step frequency" to be used for calculation of principal component analysis. In the calculation of principal component analysis, either the number of steps per second (Hz) or the number of steps per minute (spm) may be used as the numerical value of the step frequency. However, in order to prevent these multiple units from being mixed in the calculation, any one unit is used as a unified standard in the principal component analysis.

The data analysis unit 76 includes a principal component analysis unit 77 and an average value calculator 78. The principal component analysis unit 77 performs principal component analysis on the data group accumulated in the data accumulation unit 72 and stores the generated principal component analysis model in the judgment unit 80. More specifically, the principal component analysis unit 77 calculates the slope of a step frequency change and the slope of a step length change over multiple running speeds, from the data group of step frequency and step length based on the running logs of a large number of runners, and performs principal component analysis with the slope of the step frequency change as the first observed variable and the slope of the step length change as the second observed variable. The average value calculator 78 calculates an average value of the slope of the step frequency change and an average value of the slope of the step length change from the data group of step frequency and step length based on the running logs of a large number of runners and stores the average values in the judgment unit 80.

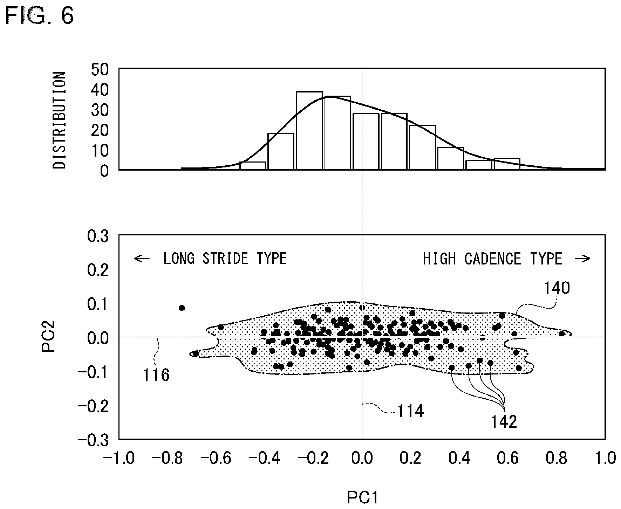

Fig. 6 shows a distribution of principal components obtained by principal component analysis. The lower part of the figure is a scatter plot in which a first principal component PC1, obtained by principal component analysis based on a data group of a large number of runners, is plotted on the horizontal axis, and a second principal component PC2, obtained by the principal component analysis, is plotted on the vertical axis. The first principal component PC1 is standardized as a value in the range of - 1.0 to 1.0 by dividing the first principal component PC1 by the maximum value of its absolute value. As shown in the lower part, a large number of data points 142 are distributed in a region 140 that is long in a horizontal axis direction. More specifically, the first principal component PC1 is distributed in a relatively wide range of - 0.75 to 0.85 across a median value of 0.0 indicated by a first dotted line 114, and the second principal component PC2 is distributed in a relatively narrow range of -0.1 to 0.1 across a median value of 0.0 indicated by a second dotted line 116. When the first principal component is larger on the negative side (in the left direction in the figure), it means that the long stride type tendency is stronger; when the first principal component is larger on the positive side (in the right direction in the figure), it means that the high cadence type tendency is stronger. The bar graph in the upper part of the figure shows that the distribution on the negative side, i.e., of the long stride type runners, is concentrated in a relatively narrow range of -0.4 to 0.0, while the distribution on the positive side, i.e., of the high cadence type runners, is dispersed in a relatively wide range of 0.0 to 0.6.

Referring back to Fig. 5, the judgment unit 80 includes a model storage unit 82, a score calculator 83, and a judgment processor 84. The model storage unit 82 stores the principal component analysis model. The principal component analysis model is generated in the form of a mathematical formula for calculating a principal component score based on principal component loading calculated by the principal component analysis unit 77 and the average value of the slope of the step frequency change and the average value of the slope of the step length change calculated by the average value calculator 78. The principal component score is standardized as a value in the range of -1.0 to 1.0 by dividing the principal component score by the maximum value of its absolute value. The mathematical formula of the principal component analysis model stored in the model storage unit 82 is as follows.

[Math. 1]

$$[SF_{Slope} \quad SL_{Slope}] \times [\text{PCA Coeff.}] - [\overline{SF_{Slope}} \quad \overline{SL_{Slope}}] = [Score_{PC1} \quad Score_{PC2}]$$

The mathematical formula 1 indicates that, by multiplying a matrix of the slope $SF_{Slope}$ of the step frequency change and the slope $SL_{Slope}$ of the step length change, newly acquired for judgment, by a rotation matrix of the principal component loading generated in advance by principal component analysis and by subtracting a matrix of the average value of the slope $SF_{Slope}$ of the step frequency change and the average value of the slope $SL_{Slope}$ of the step length change therefrom, a matrix of a first principal component score $Score_{PC1}$ and a second principal component score $Score_{PC2}$ can be obtained. Based on the principal component analysis model of the mathematical formula 1 stored in the model storage unit 82, the score calculator 83 can calculate the first principal component score $Score_{PC1}$ and the second principal component score $Score_{PC2}$, from the slope $SF_{Slope}$ of the step frequency change and the slope $SL_{Slope}$ of the step length change newly acquired.

Here, the contribution rate of the first principal component PC1 is 98.2%, and the contribution rate of the second principal component PC2 is 1.8%. Thus, the contribution rate of the first principal component PC1 is overwhelmingly higher than the contribution rate of the second principal component PC2, so that it is found that the type of the relationship between the slope of the step frequency change and the slope of the step length change, i.e., which runner type the runner belongs to, can be explained only with the first principal component PC1.

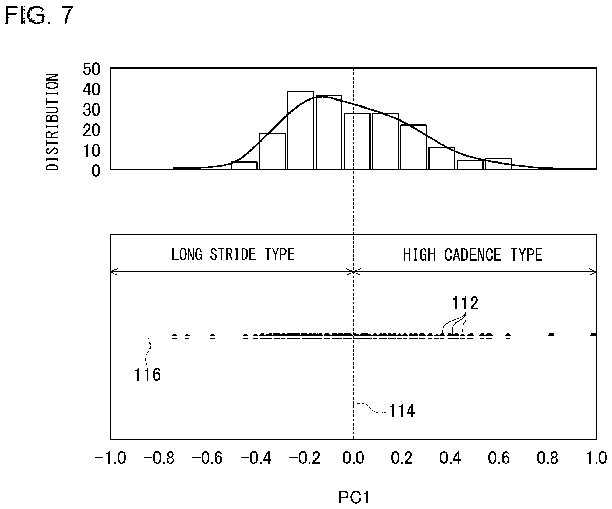

Fig. 7 shows a distribution of the first principal component obtained by principal component analysis. The lower part of the figure is a scatter plot in which the first principal component PC1 obtained by principal component analysis based on a data group of a large number of runners is plotted on the horizontal axis. Unlike the scatter plot of Fig. 6, which shows a two-dimensional distribution with the second principal component PC2 plotted on the vertical axis, this scatter plot only shows a one-dimensional distribution plotted along a horizontal axis direction. As shown in the figure, only with the distribution of the first principal component PC1, dispersion as shown in the bar graph in the upper part can be expressed. That is, it can be indicated, only using the first principal component PC1, that the larger on the negative side (in the left direction in the figure) it is, the stronger the long stride type tendency is, and, the larger on the positive side (in the right direction in the figure) it is, the stronger the high cadence type tendency is.

Thus, since the runner type can be sufficiently determined only with the first principal component PC1, dimension reduction can be performed as shown in Fig. 7; accordingly, the score calculator 83 does not need to calculate the second principal component score $Score_{PC2}$ and only calculates the first principal component score $Score_{PC1}$, and the judgment processor 84 may determine the runner type only based on the first principal component score $Score_{PC1}$. Alternatively, the score calculator 83 may calculate the first principal component score $Score_{PC1}$ and second principal component score $Score_{PC2}$, and the judgment processor 84 may determine the runner type only based on the first principal component score $Score_{PC1}$. As will be described later, an average value of 0.0 (indicated by the first dotted line 114) in a first principal component score range is used as a reference value, and, when the first principal component score $Score_{PC1}$ is 0.0 or higher, it is judged as the high cadence type; when the first principal component score $Score_{PC1}$ is 0.0 or lower, it is judged as the long stride type.

Referring back to Fig. 5, there will now be described processing for determining, based on the data of a subject newly acquired as a judgment target, the runner type of the subject. With regard to the subject's running, the step frequency acquirer 74 acquires the data of step frequency at multiple running speeds from the subject's running logs stored in the data accumulation unit 72. The step frequency acquirer 74 obtains a regression equation by regression analysis using the step frequency as the objective variable and the running speed as the explanatory variable and calculates the slope of the step frequency change based on the regression equation. The step frequency acquirer 74 performs regression analysis on at least two data points as data indicating the relationship between the running speed and the step frequency. The more data to be analyzed, the fewer the errors in the regression equation and the higher the accuracy, so that it is desirable to analyze three or more data points.

With regard to the subject's running, the step length acquirer 75 acquires the data of step length at multiple running speeds from the subject's running logs stored in the data accumulation unit 72. When the running logs do not include step length data, the step length is calculated by dividing the running distance by the step frequency. The step length acquirer 75 obtains a regression equation by regression analysis using the step length as the objective variable and the running speed as the explanatory variable and calculates the slope of the step length change based on the regression equation. The step length acquirer 75 performs regression analysis on at least two data points as data indicating the relationship between the running speed and the step length. The more data to be analyzed, the fewer the errors in the regression equation and the higher the accuracy, so that it is desirable to analyze three or more data points.

Based on the principal component analysis model stored in the model storage unit 82, the judgment unit 80 judges which of multiple running style types, including the long stride type and the high cadence type, the subject's running falls under. In the present embodiment, the running style types are classified into two types of the high cadence type and the long stride type, and it is judged which of the two is applicable. More specifically, the score calculator 83 calculates the principal component score from the slope of the step frequency change and the slope of the step length change of the subject, based on the principal component analysis model. Also, based on the principal component score, the judgment processor 84 judges whether the subject's running falls under the long stride type or the high cadence type.

The judgment processor 84 judges which of multiple running style types, including the long stride type and the high cadence type, the subject's running falls under, by using an average value in a score range as a reference and comparing the subject's principal component score with the average value. The score calculator 83 sets an average value in a score range in which scores could be calculated as the principal component score using the principal component analysis model stored in the model storage unit 82, as a reference value used to discriminate between the high cadence type and the long stride type. The average value in the score range is, for example, 0.0 indicated by the first dotted line 114 in Fig. 7. The judgment processor 84 judges that, when the first principal component score $Score_{PC1}$ of the subject is 0.0 or higher, it falls under the high cadence type, and, when the first principal component score $Score_{PC1}$ of the subject is 0.0 or lower, it falls under the long stride type. When the first principal component score $Score_{PC1}$ of the subject is equal to 0.0, the judgment processor 84 may judge that it falls under both the high cadence type and the long stride type.

Thus, as long as the slope of the step frequency change and the slope of the step length change in the subject's running can be acquired, whether the subject falls under the high cadence type or the long stride type can be judged easily and accurately. Also, as long as the principal component loading (e.g., values in a $2\times2$ matrix) obtained by principal component analysis based on the running logs of a large number of runners and an average value can be stored in advance, whether it falls under the high cadence type or the long stride type can be objectively judged only by simple calculation shown in the mathematical formula 1, with a lighter processing load. In this sense, it is also possible to calculate whether it falls under the high cadence type or the long stride type by using the user terminal 10 or a wearable device 16, without using the running style analysis server 20 for the calculation.

In the case of a method for judging the running style type based on the distribution or relative values of numerical values such as principal component scores based on principal component analysis, there is no need to prepare in advance a reference value as an absolute value, unlike a judgment method based on whether or not a measurement value of the step frequency or the step length itself exceeds a predetermined reference value. Therefore, there is no condition such that an objective reference value as an absolute value cannot be provided unless the running speed is limited to a high speed, such as a race pace at which the marathon completion time is about within 3 hours, at which the characteristics of the step frequency and the step length remarkably appear, so that the running style type can be judged for runners or measurement values of a wide range of running speeds.

The output unit 90 includes a result output unit 92, a recommendation output unit 94, and a data transmitter 96. The result output unit 92 outputs the result of the judgment by the judgment unit 80 to the user terminal 10 via the data transmitter 96. More specifically, the result output unit 92 transmits to the user terminal 10 the judgment result as to whether the subject's running style corresponds to the high cadence type or the long stride type so as to display the judgment result on the screen of the user terminal 10.

Based on the result of the judgment by the judgment processor 84, the recommendation output unit 94 determines, as recommended shoes, at least one of multiple shoes including shoes suitable for high cadence type runners and shoes suitable for long stride type runners. The recommendation output unit 94 generates and outputs information introducing shoes to be recommended. Thus, as long as the slope of the step frequency change and the slope of the step length change in the subject's running can be acquired, whether to recommend shoes suitable for high cadence type runners or shoes suitable for long stride type runners can be judged easily and accurately.

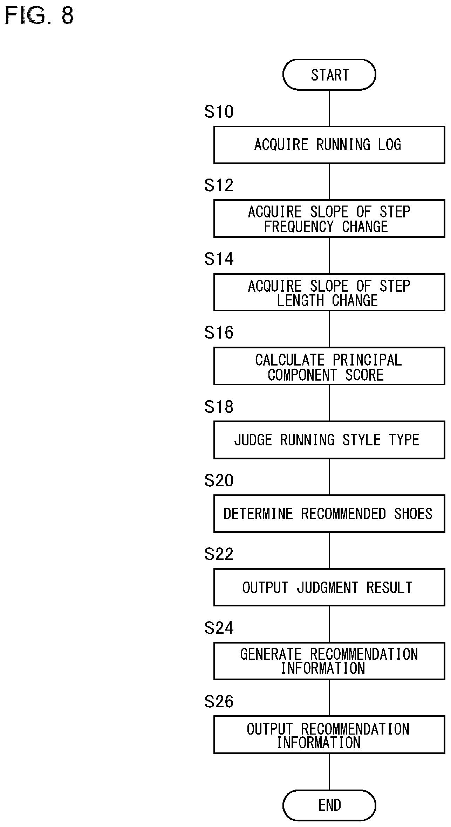

Fig. 8 is a flowchart that shows basic processing performed in the running style analysis server. The data receiver 70 acquires a running log of a subject (S10). The step frequency acquirer 74 acquires the slope of a step frequency change over multiple running speeds from the subject's running logs (S12). The step length acquirer 75 acquires the slope of a step length change over multiple running speeds from the subject's running logs (S14). The score calculator 83 calculates the principal component score from the slope of the step frequency change and the slope of the step length change of the subject, based on the principal component analysis model (S16). The judgment processor 84 judges whether the subject's running falls under the long stride type or the high cadence type, by comparing the subject's principal component score with an average value (S18). The recommendation output unit 94 determines which of multiple shoes, including shoes suitable for high cadence type runners and shoes suitable for long stride type runners, to recommend (S20). The result output unit 92 outputs the judgment result from the judgment processor 84 to the user terminal 10 (S22). The recommendation output unit 94 generates recommendation information of shoes (S24) and outputs the recommendation information to the user terminal 10 (S26).

Second Embodiment

The present embodiment differs from the first embodiment in that runners are classified into three runner types of high cadence type runners, long stride type runners, and intermediate type runners corresponding to the middle therebetween, and it is judged which of the three runner types the runner falls under and which of shoes for the three runner types is suitable, whereas, in the first embodiment, it is judged which of two runner types of the high cadence type and the long stride type the runner falls under and which of shoes for the two runner types is suitable. In the following, description will be given mainly for the differences from the first embodiment, and the explanation of features in common will be omitted.

For example, which of three running style types of the high cadence type, the long stride type, and an intermediate type corresponding to the middle therebetween is applicable may be judged as follows. That is, the judgment processor 84 judges that the high cadence type is applicable when the principal component score of the subject is within a predetermined first reference range, which is higher than an average value, judges that the long stride type is applicable when the principal component score is within a predetermined second reference range, which is lower than the average value, and judges that the intermediate type is applicable when the principal component score is within a predetermined third reference range, which is lower than the first reference range and higher than the second reference range.

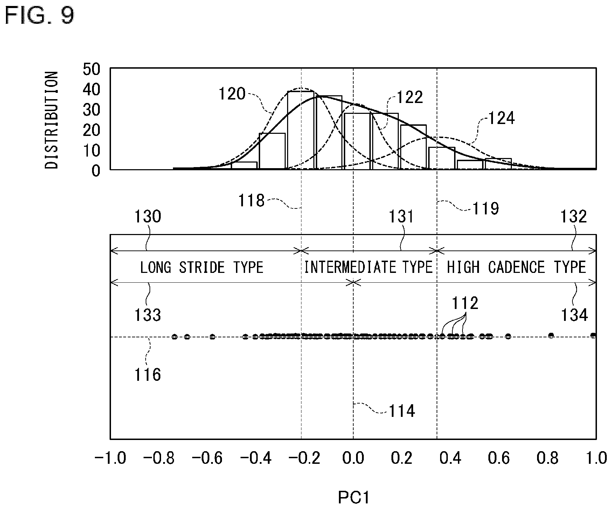

Fig. 9 shows relationships between the distribution of the first principal component obtained by principal component analysis and ranges of running style types. In the present embodiment, density estimation based on a mixed Gaussian model, in which multiple Gaussian distributions are mixed, is used. That is, it is assumed that three Gaussian distributions of high cadence type, long stride type, and intermediate type therebetween are mixed in the principal component score distribution, and, according to which one or more of the three Gaussian distributions the subject's principal component score falls under, which running style type the runner falls under is judged, and which shoes are to be recommended is determined. On the premise of the classification into the three running style types, the initial conditions of the mixed Gaussian model may be, for example, setting the vertex of a first Gaussian distribution to an average value of principal component scores corresponding to the long stride type (negative side) in Fig. 7, setting the vertex of a second Gaussian distribution to an average value of all the principal component scores (e.g., 0.0), and setting the vertex of a third Gaussian distribution to an average value of principal component scores corresponding to the high cadence type (positive side) in Fig. 7. It is assumed that each Gaussian distribution has a standard deviation with the variance of 1.0. As a result of density estimation with the above initial conditions, a long stride-type Gaussian distribution 120 with the value indicated by a third dotted line 118 as the vertex, an intermediate-type Gaussian distribution 122 with the value indicated by the first dotted line 114 as the vertex, and a high cadence-type Gaussian distribution 124 with the value indicated by a fourth dotted line 119 as the vertex are obtained.

As a method for classifying the running style types, there can be considered the case where the ranges of the three running style types are set so as not to overlap each other, and the case where the ranges of the three running style types are set so as to overlap each other. In the case where the ranges of the three running style types do not overlap each other, as shown in the figure, a first range 130, which is smaller than or equal to the value indicated by the third dotted line 118, is set as the long stride type, a second range 131 as a value range from the third dotted line 118 to the fourth dotted line 119 is set as the intermediate type, and a third range 132, which is greater than or equal to the value indicated by the fourth dotted line 119, is set as the high cadence type.

In the case where the ranges of the three running style types overlap each other, as shown in the figure, a fourth range 133, which is smaller than or equal to the value indicated by the first dotted line 114, is set as the long stride type, the second range 131 as the value range from the third dotted line 118 to the fourth dotted line 119 is set as the intermediate type, and a fifth range 134, which is greater than or equal to the value indicated by the first dotted line 114, is set as the high cadence type. In this case, when a principal component score is included in a value range from the third dotted line 118 to the first dotted line 114, the judgment processor 84 may judge that it falls under both the long stride type and the intermediate type or may judge that it falls under the intermediate type close to the long stride type. Also, when a principal component score is included in a value range from the first dotted line 114 to the fourth dotted line 119, the judgment processor 84 may judge that it falls under both the high cadence type and the intermediate type or may judge that it falls under the intermediate type close to the high cadence type. When the intermediate type close to the long stride type and the intermediate type close to the high cadence type are distinguished in the judgment, the overall classification may be made substantially into four running style types, including the long stride type and the high cadence type.

Based on the judgment result for the running style type from the judgment processor 84, the recommendation output unit 94 determines, as shoes to be recommended, one or more shoes among multiple shoes including shoes suitable for high cadence type runners, shoes suitable for long stride type runners, and intermediate type shoes suitable for both high cadence type runners and long stride type runners. However, in the case where the ranges of the three running style types do not overlap each other, as described above, the recommendation output unit 94 stores multiple shoes classified into three types of shoes suitable for the high cadence type, shoes suitable for the long stride type, and shoes suitable for the intermediate type.

On the other hand, in the case where the ranges of the three running style types overlap each other, the recommendation output unit 94 classifies shoes into two types of shoes suitable for the high cadence type and shoes suitable for the long stride type. The recommendation output unit 94 may then recommend high cadence type shoes when the running style type is judged as the high cadence type, recommend long stride type shoes when the running style type is judged as the long stride type, and recommend both the high cadence type and the long stride type when the running style type is judged as the intermediate type. Alternatively, the recommendation output unit 94 may classify shoes into three types of high cadence type shoes, long stride type shoes, and intermediate type shoes and may recommend high cadence type shoes when the running style type is judged as the high cadence type, recommend long stride type shoes when the running style type is judged as the long stride type, recommend both high cadence type shoes and intermediate type shoes when it is judged that the running style type falls under both the high cadence type and the intermediate type, and recommend both long stride type shoes and intermediate type shoes when it is judged that the running style type falls under both the long stride type and the intermediate type.

The classification method for running style types and the classification method for shoes need not necessarily be the same. For example, the running style types may be classified into two types of the high cadence type and the long stride type and which running style type is applicable may be judged, whereas shoes may be classified into three types of the high cadence type, the long stride type, and the intermediate type and which shoe type is applicable may be judged. Also, in the example shown in Fig. 9, a method is described in which multiple Gaussian distributions are estimated using density estimation based on a mixed Gaussian model, and the running style type and shoes to be recommended are determined according to which Gaussian distribution the principal component score corresponds to. However, the range of the principal component score for each running style type is to be set based on various shoe design concepts and should not necessarily be set only with statistical methods. Based on a numerical range obtained with a statistical method (such as each Gaussian distribution shown in Fig. 9), the range may be fine-tuned to set a more suitable range as the range of each running style type.

The present disclosure has been described with reference to embodiments. The embodiments are intended to be illustrative only, and it will be obvious to those skilled in the art that various modifications to a combination of constituting elements or processes could be developed and that such modifications also fall within the scope of the present disclosure. In the following, a modification will be described.

The abovementioned embodiments describe an example in which running style analysis is performed with the running style analysis system 30 including the user terminal 10 and the running style analysis server 20. In a modification, each function for the running style analysis may be implemented on a device, such as a smartphone, tablet, or personal computer, directly operated by the user, rather than on the running style analysis server 20.

Also, when the embodiments set forth above are generalized, the following aspects are obtained.

[Aspect 1]

A running style analysis device, including:

a step frequency acquirer that acquires, with regard to running of a subject, a slope of a step frequency change with respect to a change in running speed;
a step length acquirer that acquires, with regard to running of the subject, a slope of a step length change with respect to a change in running speed;
a judgment unit that calculates a principal component score from the slope of a step frequency change and the slope of a step length change of the subject, based on a principal component analysis model generated in advance based on measurement values of a plurality of runners and that makes, based on the principal component score thus calculated, judgment as to which of a plurality of running style types, including a long stride type and a high cadence type, running of the subject falls under; and
a result output unit that outputs a result of the judgment.

[Aspect 2]

The running style analysis device according to Aspect 1, wherein the judgment unit stores, as the principal component analysis model, a calculation equation for a principal component score based on principal component loading obtained by performing principal component analysis in advance on a data group of the slope of a step frequency change and the slope of a step length change with respect to a change in running speed in measurement values of a plurality of runners.

[Aspect 3]

The running style analysis device according to Aspect 2, wherein the judgment unit stores in advance an average of each of the slope of a step frequency change and the slope of a step length change with respect to a change in running speed in measurement values of a plurality of runners and stores, as the principal component analysis model, a calculation equation for calculating the principal component score by multiplying data acquired by the step frequency acquirer and the step length acquirer by the principal component loading and obtaining a difference from the average.

[Aspect 4]

The running style analysis device according to any one of Aspects 1 through 3, wherein the judgment unit judges which of a plurality of running style types, including a long stride type and a high cadence type, running of the subject falls under, by using, as a reference, an average value in a range of scores that could be calculated as a principal component score with the principal component analysis model and comparing the principal component score calculated and the average value.

[Aspect 5]

The running style analysis device according to Aspect 4, wherein the judgment unit judges that, when the principal component score is higher than or equal to the average value, the high cadence type is applicable, and, when the principal component score is lower than or equal to the average value, the long stride type is applicable.

[Aspect 6]

The running style analysis device according to Aspect 4, wherein the judgment unit judges that the high cadence type is applicable when the principal component score is within a predetermined first reference range, which is higher than the average value, judges that the long stride type is applicable when the principal component score is within a predetermined second reference range, which is lower than the average value, and judges that an intermediate type is applicable when the principal component score is within a predetermined third reference range, which is lower than the first reference range and higher than the second reference range.

[Aspect 7]

The running style analysis device according to any one of Aspects 1 through 6, further including a recommendation output unit that outputs, based on a result of the judgment, information recommending at least one of a plurality of shoes including a shoe suitable for a high cadence type runner and a shoe suitable for a long stride type runner.

[Aspect 8]

The running style analysis device according to any one of Aspects 1 through 7, further including a recommendation output unit that outputs, based on a mixed Gaussian model in which a plurality of Gaussian distributions are mixed, which each include, as a vertex, the principal component score of one of a plurality of shoes including a shoe suitable for a high cadence type runner, a shoe suitable for a long stride type runner, and a shoe suitable for both the high cadence type and the long stride type, information recommending one or more shoes among the plurality of shoes, according to which one or more of the Gaussian distributions the principal component score calculated belongs to.

[Aspect 9]

A running style analysis method, including:

acquiring, with regard to running of a subject, a slope of a step frequency change with respect to a change in running speed;

acquiring, with regard to running of the subject, a slope of a step length change with respect to a change in running speed;

calculating a principal component score from the slope of a step frequency change and the slope of a step length change of the subject, based on a principal component analysis model generated in advance based on measurement values of a plurality of runners;

making, based on the principal component score thus calculated, judgment as to which of a plurality of running style types, including a long stride type and a high cadence type, running of the subject falls under; and

outputting a result of the judgment.

[Aspect 10]

A running style analysis program causing a computer to implement:

acquiring, with regard to running of a subject, a slope of a step frequency change with respect to a change in running speed;

acquiring, with regard to running of the subject, a slope of a step length change with respect to a change in running speed;

calculating a principal component score from the slope of a step frequency change and the slope of a step length change of the subject, based on a principal component analysis model generated in advance based on measurement values of a plurality of runners and making, based on the principal component score thus calculated, judgment as to which of a plurality of running style types, including a long stride type and a high cadence type, running of the subject falls under; and

outputting a result of the judgment.

**Claims**

1.  A running style analysis device (20), comprising:

    a step frequency acquirer (74) that acquires, with regard to running of a subject, a slope of a step frequency change with respect to a change in running speed;

    a step length acquirer (75) that acquires, with regard to running of the subject, a slope of a step length change with respect to a change in running speed;

    a judgment unit (80) that calculates a principal component score from the slope of a step frequency change and the slope of a step length change of the subject, based on a principal component analysis model generated in advance based on measurement values of a plurality of runners and that makes, based on the principal component score thus calculated, judgment as to which of a plurality of running style types, including a long stride type and a high cadence type, running of the subject falls under; and

    a result output unit (90) that outputs a result of the judgment.

2.  The running style analysis device (20) according to Claim 1, wherein the judgment unit (80) stores, as the principal component analysis model, a calculation equation for a principal component score based on principal component loading obtained by performing principal component analysis in advance on a data group of the slope of a step frequency change and the slope of a step length change with respect to a change in running speed in measurement values of a plurality of runners.

3.  The running style analysis device (20) according to Claim 2, wherein the judgment unit (80) stores in advance an average of each of the slope of a step frequency change and the slope of a step length change with respect to a change in running speed in measurement values of a plurality of runners and stores, as the principal component analysis model, a calculation equation for calculating the principal component score by multiplying data acquired by the step frequency acquirer (74) and the step length acquirer (75) by the principal component loading and obtaining a difference from the average.

4.  The running style analysis device (20) according to Claim 1 or 2, wherein the judgment unit (80) judges which of a plurality of running style types, including a long stride type and a high cadence type, running of the subject falls under, by using, as a reference, an average value in a range of scores that could be calculated as a principal component score with the principal component analysis model and comparing the principal component score calculated and the average value.

5. The running style analysis device (20) according to Claim 4, wherein the judgment unit (80) judges that, when the principal component score is higher than or equal to the average value, the high cadence type is applicable, and, when the principal component score is lower than or equal to the average value, the long stride type is applicable.

6. The running style analysis device (20) according to Claim 4, wherein the judgment unit (80) judges that the high cadence type is applicable when the principal component score is within a predetermined first reference range, which is higher than the average value, judges that the long stride type is applicable when the principal component score is within a predetermined second reference range, which is lower than the average value, and judges that an intermediate type is applicable when the principal component score is within a predetermined third reference range, which is lower than the first reference range and higher than the second reference range.

7. The running style analysis device (20) according to Claim 1 or 2, further comprising a recommendation output unit (94) that outputs, based on a result of the judgment, information recommending at least one of a plurality of shoes including a shoe suitable for a high cadence type runner and a shoe suitable for a long stride type runner.

8. The running style analysis device (20) according to Claim 1 or 2, further comprising a recommendation output unit (94) that outputs, based on a mixed Gaussian model in which a plurality of Gaussian distributions are mixed, which each include, as a vertex, the principal component score of one of a plurality of shoes including a shoe suitable for a high cadence type runner, a shoe suitable for a long stride type runner, and a shoe suitable for both the high cadence type and the long stride type, information recommending one or more shoes among the plurality of shoes, according to which one or more of the Gaussian distributions the principal component score calculated belongs to.

9. A computer-implemented running style analysis method, comprising:

acquiring, with regard to running of a subject, a slope of a step frequency change with respect to a change in running speed;
acquiring, with regard to running of the subject, a slope of a step length change with respect to a change in running speed;
calculating a principal component score from the slope of a step frequency change and the slope of a step length change of the subject, based on a principal component analysis model generated in advance based on measurement values of a plurality of runners;
making, based on the principal component score thus calculated, judgment as to which of a plurality of running style types, including a long stride type and a high cadence type, running of the subject falls under; and
outputting a result of the judgment.

10. A running style analysis program causing a computer to implement:

acquiring, with regard to running of a subject, a slope of a step frequency change with respect to a change in running speed;
acquiring, with regard to running of the subject, a slope of a step length change with respect to a change in running speed;
calculating a principal component score from the slope of a step frequency change and the slope of a step length change of the subject, based on a principal component analysis model generated in advance based on measurement values of a plurality of runners and making, based on the principal component score thus calculated, judgment as to which of a plurality of running style types, including a long stride type and a high cadence type, running of the subject falls under; and
outputting a result of the judgment.

**Patentansprüche**

1. Laufstilanalysevorrichtung (20), umfassend:

ein Schrittfrequenz-Erfassungsgerät (74), das im Hinblick auf das Laufen einer Zielperson einen Anstieg der Schrittfrequenzänderung bezüglich einer Änderung der Laufgeschwindigkeit erfasst;

ein Schrittlängen-Erfassungsgerät (75), das im Hinblick auf das Laufen der Zielperson einen Anstieg einer Schrittlängenänderung bezüglich einer Änderung der Laufgeschwindigkeit erfasst;
eine Beurteilungseinheit (80), die basierend auf einem Hauptkomponentenanalysemodell, das basierend auf Messwerten einer Vielzahl von Läufern im Voraus erstellt wurde, aus dem Anstieg einer Schrittfrequenzänderung und dem Anstieg der Schrittlängenänderung der Zielperson einen Hauptkomponentenwert berechnet, und die basierend auf dem somit berechneten Hauptkomponentenwert beurteilt, unter welchen einer Vielzahl von Laufstiltypen, einschließlich eines Langschritt-Typs und eines Typs mit hoher Trittfrequenz, der Laufstil der Zielperson fällt; und
eine Ergebnisausgabeeinheit (90), die ein Ergebnis der Beurteilung ausgibt.

2. Laufstilanalysevorrichtung (20) nach Anspruch 1, wobei die Beurteilungseinheit (80) als das Hauptkomponentenanalysemodell eine Berechnungsgleichung für einen Hauptkomponentenwert speichert, der basierend auf einer Hauptkomponentenladung, die durch Durchführen einer Hauptkomponentenanalyse an einer Datengruppe des Anstiegs einer Schrittfrequenzänderung und des Anstiegs einer Schrittlängenänderung bezüglich einer Änderung der Laufgeschwindigkeit in Messwerten einer Vielzahl von Läufern im Voraus erhalten wurde.

3. Laufstilanalysevorrichtung (20) nach Anspruch 2, wobei die Beurteilungseinheit (80) im Voraus einen Mittelwert jedes des Anstiegs einer Schrittfrequenzänderung und des Anstiegs einer Schrittlängenänderung bezüglich einer Änderung der Laufgeschwindigkeit in Messwerten einer Vielzahl von Läufern speichert und als das Hauptkomponentenanalysemodell eine Berechnungsgleichung zum Berechnen des Hauptkomponentenwerts speichert, indem die von dem Schrittfrequenz-Erfassungsgerät (74) und dem Schrittlängen-Erfassungsgerät (75) erfassten Daten mit der Hauptkomponentenladung multipliziert und eine Differenz zu dem Mittelwert erhalten wird.

4. Laufstilanalysevorrichtung (20) nach Anspruch 1 oder 2, wobei die Beurteilungseinheit (80) beurteilt, unter welchen einer Vielzahl von Laufstiltypen, einschließlich eines Langschritt-Typs und eines Typs mit hoher Trittfrequenz, das Laufen der Zielperson fällt, indem ein Mittelwert in einem Bereich von Werten als Referenz verwendet wird, der als ein Hauptkomponentenwert mit dem Hauptkomponentenanalysemodell berechnet werden könnte, und den berechneten Hauptkomponentenwert mit dem Mittelwert vergleicht.

5. Laufstilanalysevorrichtung (20) nach Anspruch 4, wobei die Beurteilungseinheit (80) beurteilt, dass, wenn der Hauptkomponentenwert höher als oder gleich dem Mittelwert ist, der Typ mit hoher Trittfrequenz anwendbar ist und, wenn der Hauptkomponentenwert niedriger als oder gleich dem Mittelwert ist, der Langschritt-Typ anwendbar ist.

6. Laufstilanalysevorrichtung (20) nach Anspruch 4, wobei die Beurteilungseinheit (80) beurteilt, dass der Typ mit hoher Trittfrequenz anwendbar ist, wenn der Hauptkomponentenwert innerhalb eines vorbestimmten ersten Referenzbereichs liegt, der höher als der Mittelwert ist, beurteilt, dass der Langschritt-Typ anwendbar ist, wenn der Hauptkomponentenwert innerhalb eines vorbestimmten zweiten Referenzbereichs liegt, der niedriger als der Mittelwert ist, und beurteilt, dass ein Zwischentyp anwendbar ist, wenn der Hauptkomponentenwert innerhalb eines vorbestimmten dritten Referenzbereichs liegt, der niedriger als erste Referenzbereich und höher als der zweite Referenzbereich ist.

7. Laufstilanalysevorrichtung (20) nach Anspruch 1 oder 2, weiter umfassend eine Empfehlungsausgabeeinheit (94), die basierend auf einem Ergebnis der Beurteilung Informationen ausgibt, die mindestens einen einer Vielzahl von Schuhen empfehlen, einschließlich eines Schuhs, der für einen Läufer vom Typ mit hoher Trittfrequenz geeignet ist, und eines Schuhs, der für einen Läufer vom Langschritt-Typ geeignet ist.

8. Laufstilanalysevorrichtung (20) nach Anspruch 1 oder 2, weiter umfassend eine Empfehlungsausgabeeinheit (94), die basierend auf einem gemischten Gaußschen Modell, in dem eine Vielzahl von Gaußschen Verteilungen gemischt sind, die jeweils den Hauptkomponentenwert eines einer Vielzahl von Schuhen als einen Knotenpunkt einschließen, einschließlich eines Schuhs, der für einen Läufer vom Typ mit hoher Trittfrequenz geeignet ist, eines Schuhs, der für einen Läufer vom Langschritt-Typ geeignet ist, und eines Schuhs, der sowohl für den Typ mit hoher Trittfrequenz als auch für den Langschritt-Typ geeignet ist, Informationen ausgibt, die einen oder mehrere Schuhe aus der Vielzahl von Schuhen empfehlen, gemäß denen eine oder mehrere der Gaußschen Verteilungen zu dem berechneten Hauptkomponentenwert gehören.

9. Computerimplementiertes Laufstilanalyseverfahren, umfassend:

Erfassen eines Anstiegs einer Schrittfrequenzänderung bezüglich einer Änderung der Laufgeschwindigkeit im Hinblick auf das Laufen einer Zielperson;

Erfassen eines Anstiegs einer Schrittlängenänderung bezüglich einer Änderung der Laufgeschwindigkeit im Hinblick auf das Laufen der Zielperson;

Berechnen eines Hauptkomponentenwerts aus dem Anstieg einer Schrittfrequenzänderung und dem Anstieg einer Schrittlängenänderung der Zielperson basierend auf einem im Voraus erstellten Hauptkomponentenanalysemodell, das auf den Messwerten einer Vielzahl von Läufern basiert;

Beurteilen, basierend auf dem somit berechneten Hauptkomponentenwert, unter welchen einer Vielzahl von Laufstilen, einschließlich eines Langschritt-Typs und eines Typs mit hoher Trittfrequenz, das Laufen der Zielperson fällt; und

Ausgeben eines Ergebnisses der Beurteilung.

10. Laufstilanalyseprogramm, das einen Computer veranlasst, Folgendes zu implementieren:

Erfassen eines Anstiegs einer Schrittfrequenzänderung bezüglich einer Änderung der Laufgeschwindigkeit im Hinblick auf das Laufen einer Zielperson;

Erfassen eines Anstiegs einer Schrittlängenänderung bezüglich einer Änderung der Laufgeschwindigkeit im Hinblick auf das Laufen der Zielperson;

Berechnen eines Hauptkomponentenwerts aus dem Anstieg einer Schrittfrequenzänderung und dem Anstieg einer Schrittlängenänderung der Zielperson basierend auf einem im Voraus erstellten Hauptkomponentenanalysemodell, das auf Messwerten einer Vielzahl von Läufern basiert, und Beurteilung basierend auf dem somit berechneten Hauptkomponentenwert, unter welchen einer Vielzahl von Laufstiltypen, einschließlich eines Langschritt-Typs und eines Typs mit hoher Trittfrequenz, das Laufen der Zielperson fällt; und

Ausgeben eines Ergebnisses der Beurteilung.

## Revendications

1. Dispositif d'analyse de style de course (20), comprenant :

un acquéreur de fréquence de pas (74) qui acquiert, concernant une course d'un sujet, une pente d'un changement de fréquence de pas par rapport à un changement de vitesse de course ;

un acquéreur de longueur de pas (75) qui acquiert, concernant une course du sujet, une pente d'un changement de longueur de pas par rapport à un changement de vitesse de course ;

une unité de jugement (80) qui calcule un score de composant principal à partir de la pente d'un changement de fréquence de pas et de la pente d'un changement de longueur de pas du sujet, sur la base d'un modèle d'analyse de composant principal généré à l'avance sur la base de valeurs de mesure d'une pluralité de coureurs et qui fait un jugement, sur la base du score de composant principal ainsi calculé, à quel type, parmi une pluralité de types de style de course, incluant un type à foulée longue et un type à cadence élevée, le sujet appartient ; et

une unité d'émission de résultat (90) qui émet un résultat du jugement.

2. Dispositif d'analyse de style de course (20) selon la revendication 1, dans lequel l'unité de jugement (80) stocke, en tant que modèle d'analyse de composant principal, une équation de calcul pour un score de composant principal sur la base du chargement de composant principal obtenu en effectuant une analyse de composant principal à l'avance sur un groupe de données de la pente d'un changement de fréquence de pas et de la pente d'un changement de longueur de pas par rapport à un changement de vitesse de course dans des valeurs de mesure d'une pluralité de coureurs.

3. Dispositif d'analyse de style de course (20) selon la revendication 2, dans lequel l'unité de jugement (80) stocke à l'avance une moyenne de chacune parmi la pente d'un changement de fréquence de pas et la pente d'un changement de longueur de pas par rapport à un changement de vitesse de course dans des valeurs de mesure d'une pluralité de coureurs et stocke, comme modèle d'analyse de composant principal, une équation de calcul pour calculer le score de composant principal en multipliant des données acquises par l'acquéreur de fréquence de pas (74) et l'acquéreur de longueur de pas (75) par le chargement de composant principal et en obtenant une différence par rapport à la moyenne.

4. Dispositif d'analyse de style de course (20) selon la revendication 1 ou 2, dans lequel l'unité de jugement (80) juge à quel type, parmi une pluralité de types de style de course, incluant un type à foulée longue et un type à cadence élevée, le sujet appartient, en utilisant, comme référence, une valeur moyenne dans une plage de scores qui pourraient être calculés comme un score de composant principal avec le modèle d'analyse de composant principal et en comparant le score de composant principal calculé et la valeur moyenne.

5. Dispositif d'analyse de style de course (20) selon la revendication 4, dans lequel l'unité de jugement (80) juge que, lorsque le score de composant principal est supérieur ou égal à la valeur moyenne, le type à cadence élevée est applicable, et, lorsque le score de composant principal est inférieur ou égal à la valeur moyenne, le type à foulée longue est applicable.

6. Dispositif d'analyse de style de course (20) selon la revendication 4, dans lequel l'unité de jugement (80) juge que le type à cadence élevée est applicable lorsque le score de composant principal est au sein d'une première plage de référence prédéterminée, qui est supérieure à la valeur moyenne, juge que le type à foulée longue est applicable lorsque le score de composant principal est au sein d'une deuxième plage de référence prédéterminée, qui est inférieure à la valeur moyenne, et juge qu'un type intermédiaire est applicable lorsque le score de composant principal est au sein d'une troisième plage de référence prédéterminée, qui est inférieure à la première plage de référence et supérieure à la deuxième plage de référence.

7. Dispositif d'analyse de style de course (20) selon la revendication 1 ou 2, comprenant en outre une unité d'émission de recommandation (94) qui émet, sur la base d'un résultat du jugement, des informations recommandant au moins une chaussure parmi une pluralité de chaussures, incluant une chaussure adaptée à un coureur de type à cadence élevée et une chaussure adaptée à un coureur de type à foulée longue.

8. Dispositif d'analyse de style de course (20) selon la revendication 1 ou 2, comprenant en outre une unité d'émission de recommandation (94) qui émet, sur la base d'un modèle gaussien mixte dans lequel une pluralité de distributions gaussiennes sont mélangées, qui chacune inclut, comme sommet, le score de composant principal d'une chaussure parmi une pluralité de chaussures, incluant une chaussure adaptée à un coureur de type à cadence élevée, une chaussure adaptée à un coureur de type à foulée longue et une chaussure adaptée à la fois au type à cadence élevée et au type à foulée longue, des informations recommandant une ou plusieurs chaussures parmi la pluralité de chaussures, selon lesquelles le score de composant principal calculé appartient à une ou plusieurs des distributions gaussiennes.

9. Procédé d'analyse de style de course mis en œuvre par ordinateur, comprenant :

l'acquisition, concernant une course d'un sujet, d'une pente d'un changement de fréquence de pas par rapport à un changement de vitesse de course ;
l'acquisition, concernant une course du sujet, d'une pente d'un changement de longueur de pas par rapport à un changement de vitesse de course ;
le calcul d'un score de composant principal à partir d'une pente de changement de fréquence de pas et de la pente d'un changement de longueur de pas du sujet, sur la base d'un modèle d'analyse de composant principal généré à l'avance sur la base de valeurs de mesure d'une pluralité de coureurs ;
le jugement, sur la base du score de composant principal ainsi calculé, à quel type, parmi une pluralité de types de style de course, incluant un type à foulée longue et un type à cadence élevée, le sujet appartient ; et
l'émission d'un résultat du jugement.

10. Programme d'analyse de style de course amenant un ordinateur à mettre en œuvre :

l'acquisition, concernant une course d'un sujet, d'une pente d'un changement de fréquence de pas par rapport à un changement de vitesse de course ;
l'acquisition, concernant une course du sujet, d'une pente d'un changement de longueur de pas par rapport à un changement de vitesse de course ;
le calcul d'un score de composant principal à partir de la pente d'un changement de fréquence de pas et de la pente d'un changement de longueur de pas du sujet, sur la base d'un modèle d'analyse de composant principal généré à l'avance sur la base de valeurs de mesure d'une pluralité de coureurs et le jugement, sur la base du score de composant principal ainsi calculé, à quel type, parmi une pluralité de types de style de course, incluant un type à foulée longue et un type à cadence élevée, le sujet appartient ; et
l'émission d'un résultat du jugement.

FIG. 1

FIG. 2A

FIG. 2B

FIG. 3

FIG. 4

FIG. 5

FIG. 6

FIG. 7

FIG. 8

```
                    ( START )
                        |
S10   ┌─────────────────────────────────┐
      │       ACQUIRE RUNNING LOG        │
      └─────────────────────────────────┘
                        |
S12   ┌─────────────────────────────────┐
      │     ACQUIRE SLOPE OF STEP        │
      │       FREQUENCY CHANGE           │
      └─────────────────────────────────┘
                        |
S14   ┌─────────────────────────────────┐
      │     ACQUIRE SLOPE OF STEP        │
      │        LENGTH CHANGE             │
      └─────────────────────────────────┘
                        |
S16   ┌─────────────────────────────────┐
      │      CALCULATE PRINCIPAL         │
      │      COMPONENT SCORE             │
      └─────────────────────────────────┘
                        |
S18   ┌─────────────────────────────────┐
      │     JUDGE RUNNING STYLE TYPE     │
      └─────────────────────────────────┘
                        |
S20   ┌─────────────────────────────────┐
      │   DETERMINE RECOMMENDED SHOES    │
      └─────────────────────────────────┘
                        |
S22   ┌─────────────────────────────────┐
      │      OUTPUT JUDGMENT RESULT      │
      └─────────────────────────────────┘
                        |
S24   ┌─────────────────────────────────┐
      │    GENERATE RECOMMENDATION       │
      │         INFORMATION              │
      └─────────────────────────────────┘
                        |
S26   ┌─────────────────────────────────┐
      │    OUTPUT RECOMMENDATION         │
      │         INFORMATION              │
      └─────────────────────────────────┘
                        |
                    (  END  )
```

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4856427 B

- JP 2016214499 A